Europäisches Patentamt

European Patent Office

Office européen des brevets

(19)

(11) **EP 1 609 474 A1**

(12) **EUROPEAN PATENT APPLICATION**
published in accordance with Art. 158(3) EPC

(43) Date of publication:
28.12.2005 Bulletin 2005/52

(51) Int Cl.[7]: **A61K 33/26**, A61K 33/00,
A61K 9/16, A61K 47/30,
A61P 35/00

(21) Application number: 04720705.5

(22) Date of filing: 15.03.2004

(86) International application number:
PCT/JP2004/003397

(87) International publication number:
WO 2004/093889 (04.11.2004 Gazette 2004/45)

(84) Designated Contracting States:
AT BE BG CH CY CZ DE DK EE ES FI FR GB GR
HU IE IT LI LU MC NL PL PT RO SE SI SK TR
Designated Extension States:
AL LT LV MK

(30) Priority: 28.03.2003 JP 2003090536

(71) Applicant: Kansai Technology Licensing
Organization Co., Ltd.
Kyoto-shi, Kyoto 600-8815 (JP)

(72) Inventors:
• KOKUBO, Tadashi
Nagaokakyo-shi, Kyoto 6170841 (JP)
• KAWASHITA, Masakazu
Nagaokakyo-shi, Kyoto 6170853 (JP)
• HIRAOKA, Masahiro
Kyoto-shi, Kyoto 6060033 (JP)
• UNUMA, Hidero
Yonezawa-shi, Yamagata 9920057 (JP)

(74) Representative: Hart Davis, Jason et al
Cabinet Beau de Loménie,
158, rue de l'Université
75340 Paris Cedex 07 (FR)

(54) **METHOD FOR PREPARING THERAPEUTIC AGENT FOR CANCER**

(57) A method for the production of a material for cancer treatment, which comprises preparing a first aqueous acidic to neutral solution containing a metal to be insoluble in an alkaline solution and a carboxylic acid amide, and a second aqueous solution containing an enzyme catalyzing hydrolysis of the carboxylic acid amide and an organic polymer becoming gel by reaction with a component of the first aqueous solution or energy application from the outside, adding the second aqueous solution to the first aqueous solution, and drying. The material for cancer treatment is easy to be transported by a catheter and stay in a diseased part.

FIG.1

## Description

### TECHNICAL FIELD

The present invention relates to a method of producing a material for cancer treatment to be used for cancer treatment such as radiation therapy and thermotherapy.

### BACKGROUND ART

[Patent Document 1] JP06-62439,B
[Patent Document 2] JP02-119784,A
[Patent Document 3] JP2000-258596,A
[Non-Patent Literature Document 1] Chemical Industry, Vol.52, No.5 (2001)38-43
[Non-Patent Literature Document 2] Journal of Chemical Engineering of Japan, Vol.26, No.2 (1993)223-224
[Non-Patent Literature Document 3] New Ceramics, (1993)No.1, 47-50 [Non-Patent Literature Document 4] Annual Meeting of The Ceramic Society of Japan (2001) 270
[Non-Patent Literature Document 5] "Preparation of magnetite microspheres for hyperthermia of cancer," pp. 645-648 in Bioceramics Vol. 14, Ed. by S. Brown, I. Clarke and P. Williams, Trans Tech Publications Ltd., Switzerland, 2001

Since the medical treatment method carried out by sending microspheres made of a radioactive material to a diseased part by a catheter via a blood vessel and directly radiating radioactive beam to a cancer is capable of radiating a sufficient dose of radioactive beam to the diseased part without damaging the normal tissues in the vicinity of the body surface as compared with radiation therapy by radiating radioactive beam from the outside, its application is highly expected. Also, since a medical treatment method carried out by sending microspheres made of a ferromagnetic material to a diseased part by a catheter via a blood vessel and locally heating the diseased part by setting the diseased part in an alternating magnetic field is capable of heating the diseased part deep in vivo without damaging the normal tissues as compared with treatment by heating from the outside of the living body, the application is expected.

As the above-mentioned radioactive material, an yttria-containing glass (Patent Document No. 1) and a crystalline yttria (Patent Document No. 2) produced by high frequency induced thermal plasma method are proposed. Also, as the ferromagnetic material, magnetite-containing glass-ceramics and a magnetite crystal produced by high frequency induced thermal plasma method (both non-Patent Literature Document No. 1), and a magnetite crystal precipitated from an aqueous solution (non-Patent Literature Document No. 5) are proposed.

## DISCLOSURE OF THE INVENTION

However, the above-mentioned materials have high specific gravity values and clog a catheter and thus are hard to send prescribed amount of microspheres to a diseased part. Further, the sent microspheres descend in a body of a patient owing to the self-weight without staying in the diseased part and their distribution becomes uneven.

Therefore, an object of the invention is to provide a material for cancer treatment easy to be transported by means of a catheter and prone to stay in a diseased part.

To achieve the object, the method for the production of the material of the invention comprises preparing a first aqueous solution and a second aqueous solution; obtaining a precipitate by adding the second aqueous solution to the first aqueous solution; and drying the obtained precipitate.

The first aqueous solution is obtained by dissolving a metal becoming insoluble in an alkaline solution and a carboxylic acid amide under acidic or neutral condition.

The second aqueous solution is obtained by dissolving an enzyme catalyzing hydrolysis of the carboxylic acid amide and an organic polymer becoming gel by reaction with a component of the first aqueous solution or energy application from the outside.

Hereinafter, the case that the organic polymer becomes gel by reaction with the component of the first aqueous solution and the case that the organic polymer becomes gel by energy application from the outside will be described separately. In the case of the former, when the second aqueous solution is added to the first aqueous solution, the second aqueous solution takes the first aqueous solution therein to be gel. In the case the second aqueous solution to be added is in droplet state, gelation occurs in granular form. The carboxylic acid amide held in the gel is hydrolyzed by the function of the enzyme and the produced hydroxide ion increases the pH of the first aqueous solution. Then, the metal ionized and dissolved in the first aqueous solution is bonded with the hydroxide ion to form a precipitate. Since the above-mentioned enzyme is fixed in gel (particles), the hydrolysis is promoted only in the inside of the gel (particles) and in the vicinity of the gel (particles) to produce a precipitate reflecting the shape of the gel (particles). Since the gel (particles) is porous, the precipitate becomes porous and thus its specific gravity is small.

If the above-mentioned metal is yttrium, yttria particles having radioactive property can be produced and if iron, magnetite ($Fe_3O_4$) or maghemite ($\gamma$-$Fe_2O_3$) particle showing magnetic property can be produced. Besides, zinc, magnesium, and manganese may be contained.

If the metal is derived from a nitric acid salt, the salt is easy to be dissolved in the first aqueous solution and therefore it is preferable. The carboxylic acid amide is defined in a broad definition including urea and may include all those which are defined by the general formula $RCONH_2$. In the formula, R is not particularly limited and may be residual groups formed by removing the carboxyl group from carboxylic acid and besides, an amino group. In the case R is amino, it becomes urea.

Preferable as the above-mentioned organic polymer are an alginic acid salt and an alkyl cellulose derivative salt, because they carry out gelation by reaction with iron ion and yttrium ion, respectively.

Next, the case of carrying out gelation by energy application will be described. If the organic polymer is an albumin, gelation takes place by moderate heating and if an agar or a gelatin, gelation takes place by cooling. In the case gelation of the organic polymer is caused by the energy application from the outside, it is preferable to add the organic polymer to the first aqueous solution after previous generation by the method. Further, if the organic polymer is a pectic acid, gelation is carried out by reaction with saccharides and if a carrageenan, gelation is carried out by reaction with potassium ion under cooling condition. In these cases, the saccharides or potassium ion may be added previously in the first aqueous solution. The saccharides can be removed by firing after the precipitate is produced.

The crystallization of the precipitate is promoted and chemical durability is increased by firing the precipitate after drying and therefore, it is preferable.

Means for adding droplets of the second aqueous solution to the first aqueous solution may include dropwise addition by a dropping pipe, spraying, and also a vibration orifice method. The vibration orifice method is for jetting the second aqueous solution from an orifice with a hole diameter about several tens μm by ultrasonic vibration. According to the method, the particle diameter of the droplets can be determined based on the following expression and it is made possible to produce very small droplets with an even particle size by controlling the vibration frequency and the concentration of the second aqueous solution.

$$d = \{(6QC)/(\pi f)\}^{1/3}$$

d: particle diameter,
Q: flow speed of solution (jetting speed)
C: concentration by volume of solute in solution
f: vibration frequency (Hz)

[Effects of the invention]

As described, according to the invention, particles with a low specific gravity and having efficacious properties such as radiation and magnetic properties for medical treatment of cancer can be produced and the particles are easy to be transported by a catheter and prone to stay in a living body and thus useful for medical treatment of cancer.

**BRIEF DESCRIPTION OF THE DRAWINGS**

Fig. 1 is SEM photographs showing gel particle (after firing) of Example 1.
Fig. 2 is SEM photographs showing gel particle (after drying) of Example 2.
Fig. 3 is a SEM photograph showing gel particle (after firing) of Example 2.

**BEST MODE FOR CARRYING OUT THE INVENTION**

Example 1

A first aqueous solution was prepared by adding 0.75 g of urea to 300 mL of 0.1M yttrium nitrate (n) hydrate $Y(NO_3)_3 \cdot nH_2O$ and 10 mL of a second aqueous solution containing 1 mg of urease (derived from rapeseed) and 330 mg of carboxymethyl cellulose sodium salt was prepared.
Ten milliliters of the second aqueous solution was dropwise added by a dropping pipe to 300 mL of the first aqueous solution. Immediately after dropwise addition, the droplets independently became gel. The resulting product was left at 36 °C for 4 days and gel particles were washed successively with water and ethanol and then freeze-dried. Then, the dried gel particles were heated at 5°C/minute and fired by keeping them at respective temperatures in a range of 600 to 1300 °C for 2 hours. The diameter of the dried gel particles was about 2 to 3 mm and it became 0.5 to 1 mm after firing.

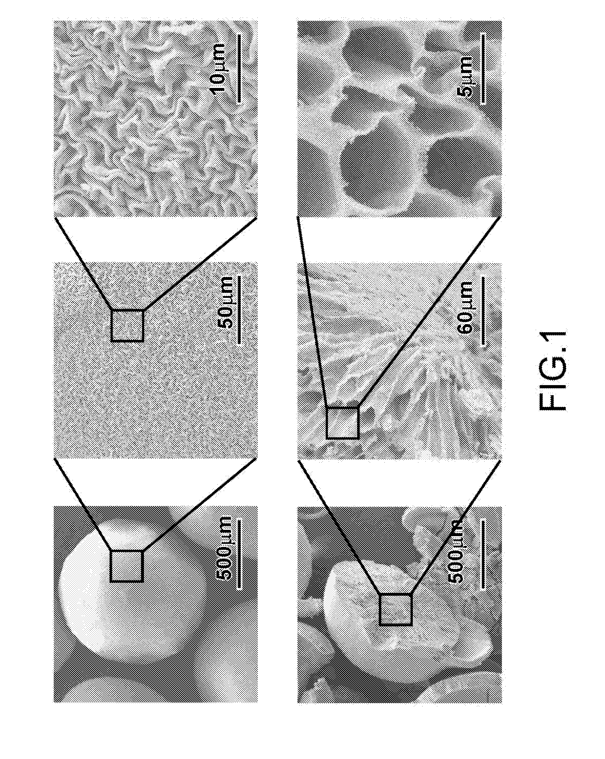

The gel particles after firing were analyzed by a powder x-ray diffractiometer to find only peaks for cubic yttrium oxide. The scanning electron microscopic (SEM) photographs of gel particles fired at 1100 °C are shown in Fig. 1. In Fig. 1, the photographs in the upper group show the outer appearance and the photographs in the lower group show a cross sectional view and the photographs in the right side in the respective groups are magnified portions of the photographs in the left side. As shown in the photographs, although no pore was formed in the surfaces of the gel particles, the insides were just like a honeycomb. That was in common among all of the gel particles fired at temperatures in a range of 600 to 1200 °C. Only the gel particles fired at 1300 °C were found having broken surfaces.
When the gel particles fired at 1000 °C were dropped in water in a beaker, it took them 3 seconds to reach the bottom in 10 cm depth from the water surface.

Example 2

A first aqueous solution containing 0.1 M iron nitrate nonahydrate $Fe(NO_3)_3 \cdot 9H_2O$ and 0.041 M urea, and 10 mL of a second aqueous solution containing 1.0 mg of urease (same as used for Example 1) and 3% by weight of ammonium alginate were prepared.
Ten milliliters of the second aqueous solution was dropwise added by a dropping pipe to 150 mL of the first aqueous solution. Immediately after dropwise addition, the droplets independently became gel. The resulting product was left at 36 °C for 3 days and gel particles were washed successively with water and ethanol and then freeze-dried. Then, the dried gel particles were heated at 5 °C/minute and fired by keeping them at 400 °C for 3 hours in $70CO_2+30H_2$ mixed gas atmosphere. The diameter of the dried gel particles was about 1.5 mm and it became about 0.5 mm after firing.

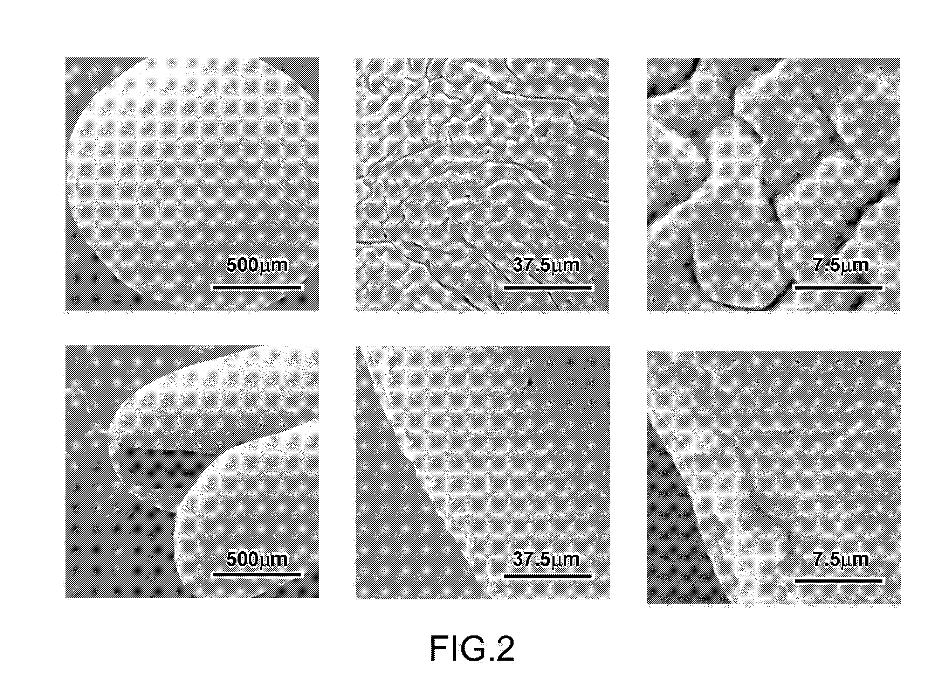

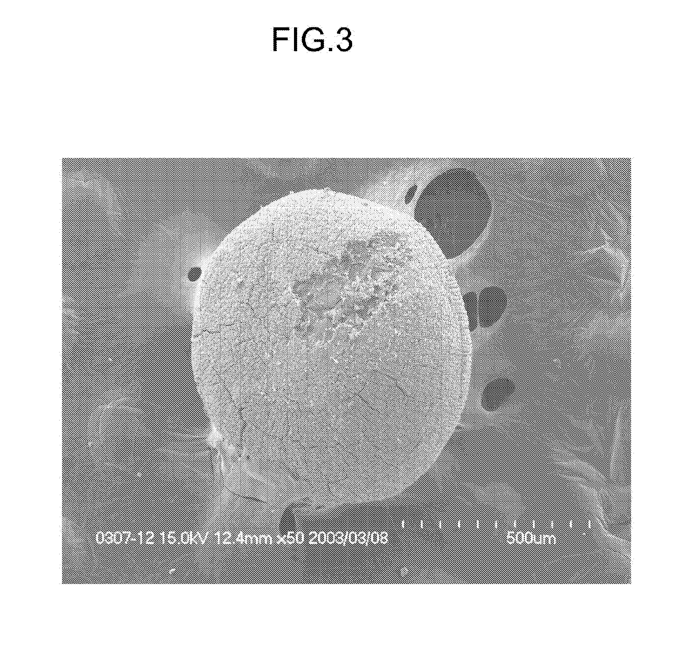

The gel particles after firing were analyzed by a powder x-ray diffractiometer to find only peaks for maghemite. The SEM photographs of the gel particles after drying are shown in Fig. 2. In Fig. 2, the photographs in the upper group show the outer appearance of gel particles and the photographs in the lower group show the outer appearance of other gel particles divided into two parts and having round shapes at the time of drying and the photographs in the right side in the respective groups are magnified portions of the photographs in the left side. Also, a SEM photograph of the gel particles after firing is shown in Fig. 3. As shown in the photographs, although no pore was formed in the outer surfaces of the gel particles, the insides were hollow.

When the fired gel particles were dropped in water in a beaker, it took them 3 seconds to reach the bottom in 10 cm depth from the water surface.

Comparative Example

A fine powder containing 99.9% by weight of yttria was melted by high frequency thermal plasma under the following conditions and made spherical.

Carrier gas for powder supply: Ar 5L/min
Plasma gas composition: Ar 90 L/min + $O_2$ 5 L/min
High frequency oscillator: plate input 40 kW, frequency 4 MHz

The spherical particles were dispersed in ultra pure water with a specific resistance 18 MΩ · cm and sieved by using a nylon sieve. Microspheres of 20 to 30 μm in diameter and containing more than 99% by weight of $Y_2O_3$ wereobtained. When the resulting microspheres were dropped in water in a beaker, it took them 1 second to reach the bottom in 10 cm depth from the water surface.

**Claims**

1. A method of producing a material for cancer treatment, the method comprising:

preparing a first aqueous solution and a second aqueous solution, the first aqueous solution containing a metal becoming insoluble in an alkaline solution and a carboxylic acid amide under acidic or neutral condition, the second aqueous solution containing an enzyme catalyzing hydrolysis of the carboxylic acid amide and an organic polymer becoming gel by reaction with a component of the first aqueous solution or energy application from the outside;
obtaining a precipitate by adding the second aqueous solution to the first aqueous solution; and
drying the obtained precipitate.

2. The method according to claim 1, wherein the second aqueous solution is added in droplet state to the first aqueous solution

3. The method according to claim 1 or 2, wherein the metal is at least one selected from yttrium and iron.

4. The method according to any one of claims 1 to 3, wherein the metal is derived from a nitric acid salt.

5. The method according to any one of claims 1 to4, wherein the organic polymer is at least one selected from the group consisting of an alginic acid salt, an alkyl cellulose derivative salt, an albumin, a pectic acid, a carrageenan, an agar and a gelatin.

6. The method according to any one of claims 1 to5, further comprising firing the precipitate after drying.

FIG.1

FIG.2

# FIG.3

0307-12 15.0kV 12.4mm x50 2003/03/08    500um

<div align="center">

**INTERNATIONAL SEARCH REPORT**

</div>

| | International application No. |
|---|---|
| | PCT/JP2004/003397 |

| A. CLASSIFICATION OF SUBJECT MATTER |
|---|
| Int.Cl$^7$  A61K33/26, 33/00, 9/16, 47/30, A61P35/00 |

According to International Patent Classification (IPC) or to both national classification and IPC

| B. FIELDS SEARCHED |
|---|
| Minimum documentation searched (classification system followed by classification symbols)<br>  Int.Cl$^7$  A61K33/26, 33/00, 9/16, 47/30 |
| Documentation searched other than minimum documentation to the extent that such documents are included in the fields searched |
| Electronic data base consulted during the international search (name of data base and, where practicable, search terms used) |

| C. DOCUMENTS CONSIDERED TO BE RELEVANT |

| Category* | Citation of document, with indication, where appropriate, of the relevant passages | Relevant to claim No. |
|---|---|---|
| A | JP 02-119784 A  (Meiji Seika Kaisha, Ltd.),<br>07 May, 1990 (07.05.90),<br>Full text; particularly. examples 4, 7<br>(Family: none) | 1-6 |
| A | WO 89/01521 A1  (Shingijutsu Kaihatsu Jigyodan),<br>23 February, 1989 (23.02.89),<br>& US 4945049 A          & DE 3890648 A | 1-6 |
| A | JP 09-508897 A  (Bruce Nathaniel Gray),<br>09 September, 1997 (09.09.97),<br>& WO 95/19841 A1        & EP 740581 A1<br>& US 5885547 A        & AU 9515279 A | 1-6 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>  08 June, 2004 (08.06.04) | Date of mailing of the international search report<br>  06 July, 2004 (06.07.04) |
|---|---|
| Name and mailing address of the ISA/<br>  Japanese Patent Office | Authorized officer |
| Facsimile No. | Telephone No. |

Form PCT/ISA/210 (second sheet) (January 2004)

**EP 1 609 474 A1**

<table>
<tr><td colspan="3"><strong>INTERNATIONAL SEARCH REPORT</strong></td><td colspan="2">International application No.<br>PCT/JP2004/003397</td></tr>
</table>

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 2002-538616 A (Paragon Medical Ltd.), 12 November, 2002 (12.11.02), & WO 2000/052714 A1 & EP 1166291 A1 & US 6599234 B1 | 1-6 |

Form PCT/ISA/210 (continuation of second sheet) (January 2004)

9